# EUROPEAN PATENT SPECIFICATION

(11) **EP 3 500 159 B1**
(45) Date of publication and mention of the grant of the patent: **05.06.2024**
(21) Application number: 17841842.2
(22) Date of filing: 02.08.2017
(51) Int. Cl.: A61B 1/267, A61B 5/06, A61B 5/11, A61B 10/02, A61B 34/20, G06T 7/246, A61B 5/113, A61B 17/00, A61B 34/10, A61B 34/00

(54) **SYSTEM FOR THE USE OF SOFT-POINT FEATURES TO PREDICT RESPIRATORY CYCLES AND IMPROVE END REGISTRATION**
SYSTEM ZUR VERWENDUNG VON WEICHPUNKTMERKMALEN ZUR VORHERSAGE VON ATMUNGSZYKLEN UND ZUR VERBESSERUNG DER ENDREGISTRIERUNG
SYSTÈME POUR L'UTILISATION DE CARACTÉRISTIQUES DE POINTS MOUS POUR PRÉDIRE DES CYCLES RESPIRATOIRES ET AMÉLIORER L'ENREGISTREMENT FINAL

(30) Priority: 17.08.2016 US 201615238905
(43) Date of publication of application: 26.06.2019
(73) Proprietor: Covidien LP, Mansfield, MA 02048 (US)
(72) Inventor: KRIMSKY, William S., Forest Hill, MD 21050 (US)
(74) Representative: Maschio & Soames IP Ltd
(86) International application number: PCT/US2017/045110
(87) International publication number: WO 2018/034845

(56) References cited:
- EP-A1- 2 732 765
- EP-A1- 2 953 532
- WO-A1-2015/103061
- WO-A1-2016/018648
- WO-A2-2015/164587
- US-A1- 2005 182 295
- US-A1- 2014 177 931
- US-A1- 2015 073 268
- US-A1- 2015 265 257
- US-A1- 2015 265 368
- US-A1- 2016 135 664
- Pall J Reynisson ET AL: "Navigated bronchoscopy: a technical review", Journal of bronchology & interventional pulmonology, vol. 21, no. 3 1 July 2014 (2014-07-01), pages 242-264, XP055272084, United States DOI: 10.1097/LBR.0000000000000064 Retrieved from the Internet: URL:https://www.researchgate.net/publicati on/263708601_Navigated_Bronchoscopy_A_Tech nical_Review [retrieved on 2016-05-12]
- J.R. MCCLELLAND ET AL: "Respiratory motion models: A review", MEDICAL IMAGE ANALYSIS, vol. 17, no. 1, 1 January 2013 (2013-01-01), pages 19-42, XP055106223, ISSN: 1361-8415, DOI: 10.1016/j.media.2012.09.005

## Description

### BACKGROUND

### Technical Field

The present disclosure relates to modeling movement with an area of interest of a patient's body and, more particularly, to devices, systems, and methods for automatically registering and updating a three-dimensional model of the area of interest, with a patient's real features, throughout a breathing cycle.

### Description of Related Art

A common device for inspecting the airway of a patient is a bronchoscope. Typically, the bronchoscope is inserted into a patient's airways through the patient's nose or mouth and can extend into the lungs of the patient. A typical bronchoscope includes an elongated flexible tube having an illumination assembly for illuminating the region distal to the bronchoscope's tip, an imaging assembly for providing a video image from the bronchoscope's tip, and a working channel through which instruments, e.g., diagnostic instruments such as biopsy tools, therapeutic instruments can be inserted.

Bronchoscopes, however, are limited in how far they may be advanced through the airways due to their size. Where the bronchoscope is too large to reach a target location deep in the lungs, a clinician may utilize certain real-time imaging modalities such as fluoroscopy. Fluoroscopic images, while useful, present certain drawbacks for navigation as it is often difficult to distinguish luminal passageways from solid tissue. Moreover, the images generated by the fluoroscope are two-dimensional whereas navigating the airways of a patient requires the ability to maneuver in three dimensions.

To address these issues, systems have been developed that enable the development of three-dimensional models of the airways or other luminal networks, typically from a series of computed tomography (CT) images. One such system has been developed as part of the ILOGIC^{®} ELECTROMAGNETIC NAVIGATION BRONCHOSCOPY^{®} (ENB^{™}), system currently sold by Medtronic PLC. The details of such a system are described in commonly assigned U.S. Patent No. 7,233,820, entitled ENDOSCOPE STRUCTURES AND TECHNIQUES FOR NAVIGATING TO A TARGET IN BRANCHED STRUCTURE, filed on March 29, 2004, by Gilboa, and commonly assigned U.S. Patent Application Serial No. 13/836,203, entitled SYSTEM AND METHOD FOR NAVIGATING WITHIN THE LUNG, by Brown.

While the system as described in U.S. Patent No. 7,233,820 is quite capable, there is always a need for development of improvements and additions to such systems US 2005/182295 A1 discloses a system for visual-assisted guidance of an ultra-thin flexible endoscope to a predetermined region of interest within a lung during a bronchoscopy procedure. The region may be an opacity-identified by non-invasive imaging methods, such as high-resolution computed tomography (HRCT) or as a malignant lung mass that was diagnosed in a previous examination. An embedded position sensor on the flexible endoscope indicates the position of the distal tip of the probe in a Cartesian coordinate system during the procedure. A visual display is continually updated, showing the present position and orientation of the marker in a 3-D graphical airway model generated from image reconstruction. The visual display also includes windows depicting a virtual fly-through perspective and real-time video images acquired at the head of the endoscope, which can be stored as data, with an audio or textual account.

### SUMMARY

The invention is defined by appended claim 1.

### BRIEF DESCRIPTION OF THE DRAWINGS

Various aspects and features of the present disclosure are described herein below with references to the drawings, wherein:
FIG. 1 is a perspective view of an electromagnetic navigation system in accordance with the present disclosure;
FIG. 2 is a flowchart illustrating a method of using soft points to improve registration of a luminal network to a model of the luminal network, provided in accordance with and embodiment of the present disclosure
FIG. 3 is a flowchart illustrating a method of using soft points and tidal volume calculations to improve registration of a luminal network to a model of the luminal network, provided in accordance with and embodiment of the present disclosure.
FIG. 4 is yet a flowchart illustrating a method of using soft points and static points to improve registration of a luminal network to a model of the luminal network, provided in accordance with and embodiment of the present disclosure.
FIG. 5A is a graphical illustration of the target management mode in accordance with embodiments of the present disclosure;
FIG. 5B is a subsequent graphical illustration of the target management mode in accordance with embodiments of the present disclosure after a ;
FIG. 6 is an illustration of a user interface of the workstation of FIG. 7 presenting a view for performing navigation to a target further presenting a central navigation tab;
FIG. 7 is a schematic diagram of a workstation configured for use with the system of FIG. 1.

### DETAILED DESCRIPTION

The present disclosure is directed to devices, systems, and methods for performing localized registration of a bronchial tree to improve an initial registration and better depict a patient's airways and lung movement due to patient breathing. The localized registration methods of the present disclosure involve navigating a sensor to a soft point target, confirming the location of the sensor with an imaging system, and initiating a tracking protocol to track the location of the sensor over a period of time, such as a period encompassing a breathing cycle. The tracked location of the sensor over time allows a localized registration of various points with respect to a previously imaged and previously model registration of a bronchial tree.

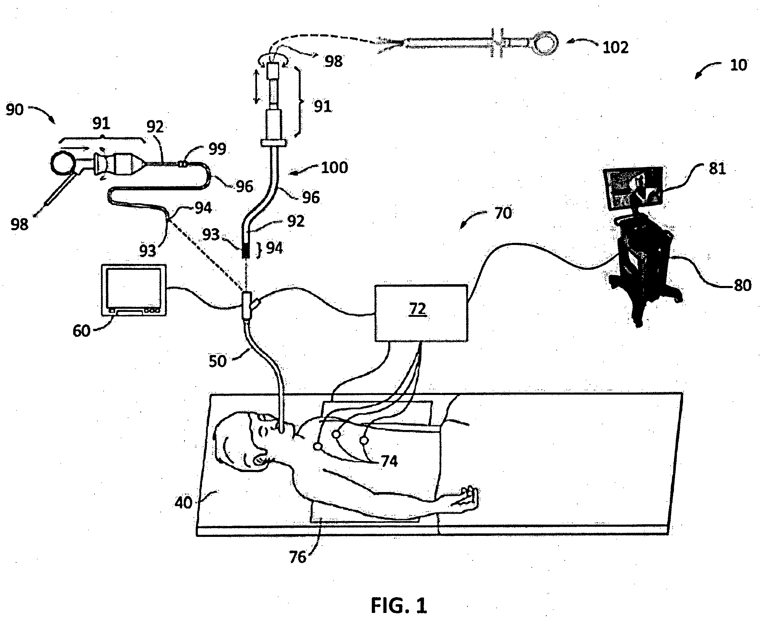

With reference to FIG. 1, an electromagnetic navigation (EMN) system 10 is provided in accordance with the present disclosure. Among other tasks that may be performed using the EMN system 10 are planning a pathway to target tissue, navigating a catheter assembly to the target tissue, navigating a biopsy tool or treatment tool, such as an ablation catheter, to the target tissue to obtain a tissue sample from the target tissue using the biopsy tool, digitally marking the location where the tissue sample was obtained, and placing one or more echogenic markers at or around the target.

EMN system 10 generally includes an operating table 40 configured to support a patient; a bronchoscope 50 configured for insertion through the patient's mouth and/or nose into the patient's airways; monitoring equipment 60 coupled to bronchoscope 50 for displaying video images received from bronchoscope 50; a tracking system 70 including a tracking module 72, a plurality of reference sensors 74, and an electromagnetic field generator 76; a workstation 80 including software and/or hardware used to facilitate pathway planning, identification of target tissue, navigation to target tissue, and digitally marking the biopsy location
FIG. 1 also depicts two types of catheter guide assemblies 90, 100. Both catheter guide assemblies 90, 100 are usable with EMN system 10 and share a number of common components. Each catheter guide assembly 90, 100 includes a handle 91, which is connected to an extended working channel (EWC) 96. EWC 96 is sized for placement into the working channel of a bronchoscope 50. In operation, a locatable guide (LG) 92, including an electromagnetic (EM) sensor 94, is inserted into EWC 96 and locked into position such that EM sensor 94 extends a desired distance beyond a distal tip 93 of EWC 96. The location of EM sensor 94, and thus the distal end of EWC 96, within an electromagnetic field generated by electromagnetic field generator 76 can be derived by tracking module 72, and workstation 80. Catheter guide assemblies 90, 100 have different operating mechanisms, but each contain a handle 91 that can be manipulated by rotation and compression to steer distal tip 93 of LG 92 and EWC 96. Catheter guide assemblies 90 are currently marketed and sold by Covidien LP under the name SUPERDIMENSION^{®} Procedure Kits. Similarly, catheter guide assemblies 100 are currently sold by Covidien LP under the name EDGE^{™} Procedure Kits. Both kits include a handle 91, EWC 96, and LG 92. For a more detailed description of the catheter guide assemblies 90, 100, reference is made to commonly-owned U.S. Patent Application Serial No. 13/836,203 entitled MICROWAVE ABLATION CATHETER AND METHOD OF UTILIZING THE SAME, filed on March 15, 2013, by Ladtkow et al.

As illustrated in FIG. 1, the patient is shown lying on operating table 40 with bronchoscope 50 inserted through the patient's mouth and into the patient's airways. Bronchoscope 50 includes a source of illumination and a video imaging system (not explicitly shown) and is coupled to monitoring equipment 60, e.g., a video display, for displaying the video images received from the video imaging system of bronchoscope 50.

Catheter guide assemblies 90, 100 including LG 92 and EWC 96 are configured for insertion through a working channel of bronchoscope 50 into the patient's airways (although the catheter guide assemblies 90, 100 may alternatively be used without bronchoscope 50). LG 92 and EWC 96 are selectively lockable relative to one another via a locking mechanism 99. A six degrees-of-freedom electromagnetic tracking system 70, e.g., similar to those disclosed in U.S. Patent No. 6,188,355, entitled WIRELESS SIX-DEGREE-OF-FREEDOM LOCATOR, filed on December 14, 1998, by Gilboa, and published PCT Application Nos. WO 2000/10456 entitled INTRABODY NAVIGATION SYSTEM FOR MEDICAL APPLICATIONS, filed on July 7, 1999, by Gilboa et al. and WO 2001/67035, entitled OBJECT TRACKING USING A SINGLE SENSOR OR A PAIR OF SENSORS, filed on September 3, 2001, by Gilboa et al., or any other suitable positioning measuring system, is utilized for performing navigation, although other configurations are also contemplated. Tracking system 70 is configured for use with catheter guide assemblies 90, 100 to track the position of EM sensor 94 as it moves in conjunction with EWC 96 through the airways of the patient, as detailed below.

As shown in FIG. 1, electromagnetic field generator 76 is positioned beneath the patient. Electromagnetic field generator 76 and the plurality of reference sensors 74 are interconnected with tracking module 72, which derives the location of each reference sensor 74. One or more of reference sensors 74 are attached to the chest of the patient. One or more reference sensors 74 may also be attached to a plurality of locations including those at static points such as i.e. a vertebral body, a main carina, sternum, thyroid cartilage, rib, an esophagus, etc. or at soft points such as i.e. a nipple line, an esophagus, a rib outline, a secondary carina, etc.
The coordinates of reference sensors 74 are sent to workstation 80, which includes and application 81 which uses data collected by sensors 74 to calculate a patient coordinate frame of reference.

Also shown in FIG. 1 is a catheter biopsy tool 102 that is insertable into catheter guide assemblies 90, 100 following navigation to a target and removal of LG 92. Biopsy tool 102 is used to collect one or more tissue samples from the target tissue. As detailed below, biopsy tool 102 is further configured for use in conjunction with tracking system 70 to facilitate navigation of biopsy tool 102 to the target tissue, tracking of a location of biopsy tool 102 as it is manipulated relative to the target tissue to obtain the tissue sample, and/or marking the location where the tissue sample was obtained.

Although navigation is detailed above with respect to EM sensor 94 being included in LG 92 it is also envisioned that EM sensor 94 may be embedded or incorporated within biopsy tool 102 where biopsy tool 102 may alternatively be utilized for navigation without need of LG 92 or the necessary tool exchanges that use of LG 92 requires. A variety of useable biopsy tools are described in Pub. Nos. US 2015/0141869 and US 2015/0265257 both entitled DEVICES, SYSTEMS, AND METHODS FOR NAVIGATING A BIOPSY TOOL TO A TARGET LOCATION AND OBTAINING A TISSUE SAMPLE USING THE SAME, filed May 21, 2015 and September 24, 2015, respectively, by Costello et al., and in Pub. No. WO2015076936having the same title and filed September 30, 2014, by Costello et al. and useable with EMN system 10 as described herein.

During procedure planning, workstation 80 utilizes computed tomographic (CT) image data for generating and viewing the 3D model of the patient's airways, enables the identification of target tissue on the 3D model (automatically, semiautomatically or manually), and allows for the selection of a pathway through the patient's airways to the target tissue. More specifically, the CT scans are processed and assembled into a 3D volume, which is then utilized to generate the 3D model of the patient's airways. The 3D model may be presented on a display monitor associated with workstation 80, or in any other suitable fashion. Using workstation 80, various slices of the 3D volume and views of the 3D model may be presented and/or may be manipulated by a clinician to facilitate identification of a target and selection of a suitable pathway through the patient's airways to access the target. The 3D model may also show marks of the locations where previous biopsies were performed, including the dates, times, and other identifying information regarding the tissue samples obtained. These marks may also be selected as the target to which a pathway can be planned. Once selected, the pathway is saved for use during the navigation procedure. An example of a suitable pathway planning system and method is described in Pub. Nos. US 2014/0281961; US 2014/0270441; and 2014/0282216, all entitled PATHWAY PLANNING SYSTEM AND METHOD, filed on March 15, 2014, by Baker. During navigation, EM sensor 94, in conjunction with tracking system 70, enables tracking of EM sensor 94 and/or biopsy tool 102 as EM sensor 94 or biopsy tool 102 is advanced through the patient's airways.

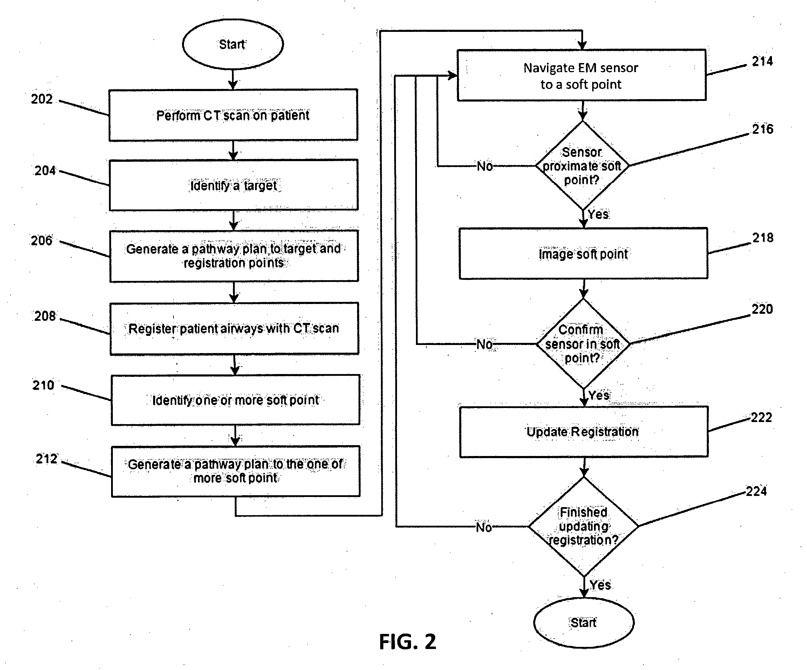

Referring now to FIG. 2, there is shown a flowchart of an example method for updating the registration of the 3D model with a patient's airways. As described above, at step 202, an area of interest, for instance the chest and lungs, of a patient is imaged using imaging methods such as, for example, a CT scan. At step 204, a target is identified in the images generated in step 202. Once a target is established, at step 206, a path through the branches of the airways to the target is generated in the CT image data. Once the pathway plan has been developed and is accepted by the clinician, the pathway plan can be utilized in a navigation procedure using the EMN system 10. The pathway plan is loaded into an application on workstation 80 and displayed. Then, at step 208, application 81 performs the registration of the CT scan with the patient's airways, as described above, and in particular as described in co-pending U.S. Patent Application No. 14/790,581, entitled REAL TIME AUTOMATIC REGISTRATION FEEDBACK, filed on July 2, 2015, by Brown et al. During registration, the location of EM sensor 94 within the patient's airways is tracked, and a plurality of points denoting the location of EM sensor 94 within the EM field generated by EM generator 76 is generated. When sufficient points have been collected, the application 81 compares the locations of these points to the 3D model and seeks to fit all the points within the lumens of the 3D model. When a fit is established, signifying that the majority if not all of the points have been fit within the area defined by the 3D model of the airway, the patient and the 3D model are registered to one another. As a result, detected movement of the EM sensor 94 within the patient can be accurately depicted on the display of the workstation 80 as a sensor 94 traversing the 3D model or a 2D image from which the 3D model was generated.

At step 210, a physician or application 81 may identify one or more soft point targets, e.g., a nipple line, an esophagus, a rib outline, a secondary carina, etc. Once a soft point is established, at step 212, a path to the target is generated in the CT image data by Application 81. The path may provide guidance for navigation of the EM sensor through the bronchial network of the lung to or near the soft point. The path may then further provide for the EM sensor to be guided from a location near the soft point, through a bronchial wall of the lungs to a soft point located outside of, but near the bronchial tree. In the alternative, the path may provide guidance for the EM sensor to be inserted percutaneously through the patient's skin to the location of the soft point with or without additional guidance through the bronchial tree. After the pathway plan has been developed and is accepted by the clinician, the pathway plan can be utilized in a navigation procedure using the EMN system 10. Application 81 begins navigation process, at step 214 by displaying guidance for navigating EM sensor proximate to a soft point target, such as i.e. a nipple line, an esophagus, a rib outline, a secondary carina, etc., while tracking the location of EM sensor 94. In the alternative, by viewing a live video feed from a camera located proximate EM sensor 94 (e.g., in a bronchoscope) a soft point target may be detected visually by a clinician. Thereafter, at step 216, the clinician or application 81 may determine whether the sensor is located proximate to a determined soft point target. Unless the clinician or application 81 determines that EM sensor 94 is proximate a soft point target, processing returns to step 214 where further guidance is displayed.

At step 218, the soft point target is imaged while EM sensor 94 is located proximate the soft point using, for example, CT imaging, cone beam CT imaging, or ultrasonic imaging. Using the image generated in step 218, at step 220, a clinician or application 81 confirms EM sensor's 94 location at the soft point. If it is determined that the EM sensor 94 is not at the soft point target, processing returns to step 214 where further guidance is displayed. If EM sensor 94 is confirmed to be proximate the soft point, processing proceeds to step 222.

At step 222, application 81 uses the stored points denoting the location of EM sensor 94 to perform localized registration to update the 3D model with the patient's airways proximate the soft point target. For example, localized registration may be performed based on a range of interpolation techniques, such as Thin Plates Splines (TPS) interpolation. In embodiments, TPS interpolation may be used for non-rigid registration of the points denoting the location of EM sensor 94 within the EM field generated by EM generator 76 stored during automatic registration with the 3D model, and may be augmented by additional points stored during localized registration.

At step 224, application 81 or a clinician determines updating registration to be complete if there are no remaining soft point targets for which localized registration is to be performed. If updating registration is not complete, processing returns to step 214, where application 81 displays guidance for navigating EM sensor 94 proximate the next soft point target. If updating registration is complete, the processing ends.

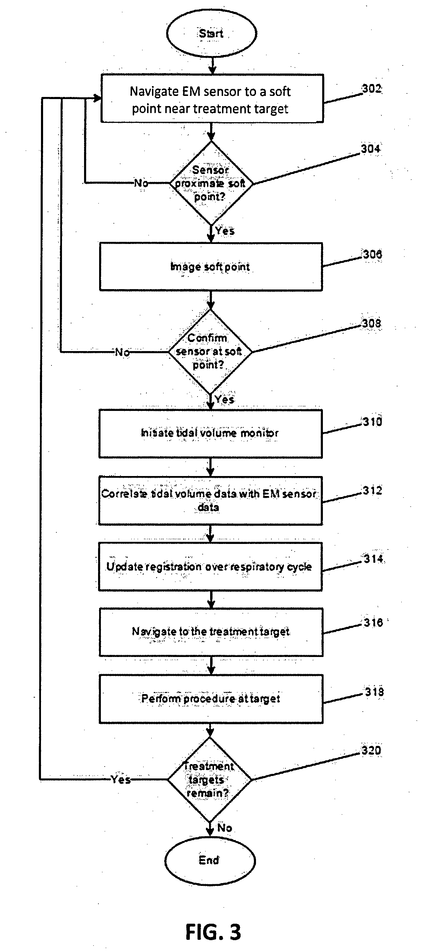

Turning to FIG. 3, there is shown another flowchart of an example method for updating a registration of the 3D model with a patient's airways. At step 302, application 81 displays guidance for navigating, through the a luminal network, through a wall of a luminal network, or percutaneously through a patient's skin, EM 94 sensor proximate to a soft point target, such as i.e. a nipple line, an esophagus, a rib outline, a secondary carina, etc., near a treatment target, while tracking the location of EM sensor 94. In the alternative, by viewing a live video feed from a camera located proximate EM sensor 94 (e.g., in a bronchoscope) a soft point target may be detected visually by the clinician. Thereinafter, at step 304, a clinician or application 81 determines whether EM sensor 94 is proximate to the soft point target. If no, processing returns to step 302, where application 81 resumes displaying guidance for navigating EM sensor 94 proximate the soft point target. If yes, processing proceeds to step 306.

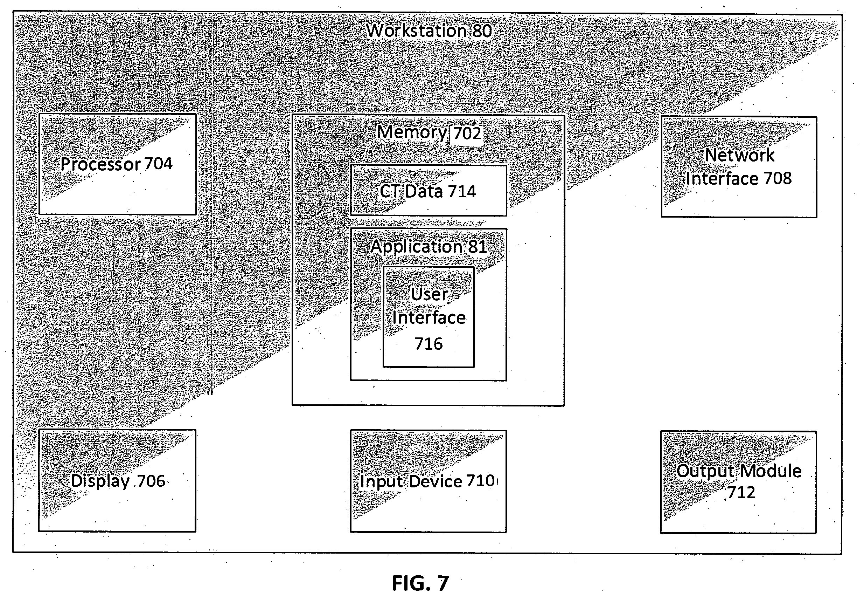

At step 306, the soft point target is imaged while EM sensor 94 is located proximate the soft point using, for example, CT imagining, cone beam CT imaging, or ultrasonic imaging. Using the image generated in step 306, at step 308, a clinician or application 81 may confirm EM sensor's 94 location at the soft point. In confirming whether EM sensor 94 is located at the soft point, the image generated in step 406 may be displayed on display 706 (FIG. 7). If it is determined that the EM sensor 94 is not at the soft point target, processing returns to step 302 where further guidance is displayed. If EM sensor 94 is confirmed to be proximate the soft point, processing proceeds to step 310.

At step 310, the movement of the patient's chest caused by tidal volume breathing is sampled throughout one or more cycles of the patient's breathing cycle. Movement caused by tidal volume breathing may be sampled using one or more optical cameras positioned to view and record the movement of the patient's chest. The movement of the patient's chest may be used to estimate the movement caused by tidal breathing. In the alternative, sensors 74 may be sampled to determine the movement of the patient's chest during the patient's tidal breathing. The movement of the patient's chest sensed using sensors 74 similarly maybe be used to estimate the movement cause by tidal breathing.

At step 312, application 81 receives the patient's tidal volume movement data and location data from EM sensor 94 and correlates the data sets. By correlating the data sets, the present disclosure seeks to apportion the observed chest movement to movement of the EM sensor 94. That is, if the chest is observed moving a distance in one direction (e.g., normal to the longitudinal axis of the spine) a determination can be made as to the magnitude of the movement that could be observed in the airway of the lungs proximate EM sensor 94. Application 81 saves the data and correlates the patient's volume breathing movement data and location data from EM sensor 94 according to the time the data points were received.

The saved data may be transferred and saved to a larger database and conglomerated with similar saved data from other patients in order to be utilized in future procedures. The database also includes additional factors of each patient such as height, weight, sex, gender, peak expiratory flow rate, and forced expiratory volume. By analyzing the saved data of many patients saved on the database, a predictive model may be generated to determine a likely location of a target within the lungs or to update a model of the patient's lungs without performing an invasive procedure. The predictive model may further incorporate additional factors to create a comprehensive estimation of movement throughout the breathing cycle.

In practice, a physician performs a CT scan on a patient's lungs and generates a model. Then, the physician measures movement caused by tidal volume breathing using, for example, one or more optical cameras positioned to view and record the movement of the patient's chest, and generates data. Finally, the measured movement data and patient's additional factors are input into the predictive model in order to generate a predicted estimation of points within the lungs or to improve the model generated in the CT scan throughout the breathing cycle.

At step 314, application 81 uses the correlated tidal movement volume data and EM sensor 94 location data to perform localized registration to update the 3D model with the patient's airways proximate the soft point target. For example, localized registration may be performed based on a range of interpolation techniques, such as Thin Plates Splines (TPS) interpolation. In embodiments, TPS interpolation may be used for non-rigid registration of the points denoting the location of EM sensor 94 within the EM field generated by EM generator 76 stored during automatic registration with the 3D model, and may be augmented by additional points stored during localized registration. As a result of the correlation in step 312 and the registration updating in step 314, the detected movement of the EM sensor 94, which otherwise would be depicted on the display of static CT images, or the 3D model derived therefrom, is modified to more accurately display the location of the EM sensor 94 and any tool it is operatively connected to within the airways of the patient. Without such correlation and localized registration, the detected location of the EM sensor 94 can appear to be outside of the airways of the patient during certain portions of the patient's breathing cycle.

At step 316, the updated registration is incorporated into the model and guidance is display to enable a physician to navigate to the treatment target. Upon reaching the treatment target, at step 318, a procedure is performed. The updated registration provides a more accurate representation of the location of the treatment target. All updates to the registration are performed as background processes as the user only view the results of the updated registration.

At step 320, application 81 or a clinician determines if there are additional treatment targets. If treatment targets remain, processing returns to step 302, where application 81 displays guidance for navigating EM sensor 94 proximate the next soft point near the next treatment target. If no treatment targets remain, the process is complete and processing ends.

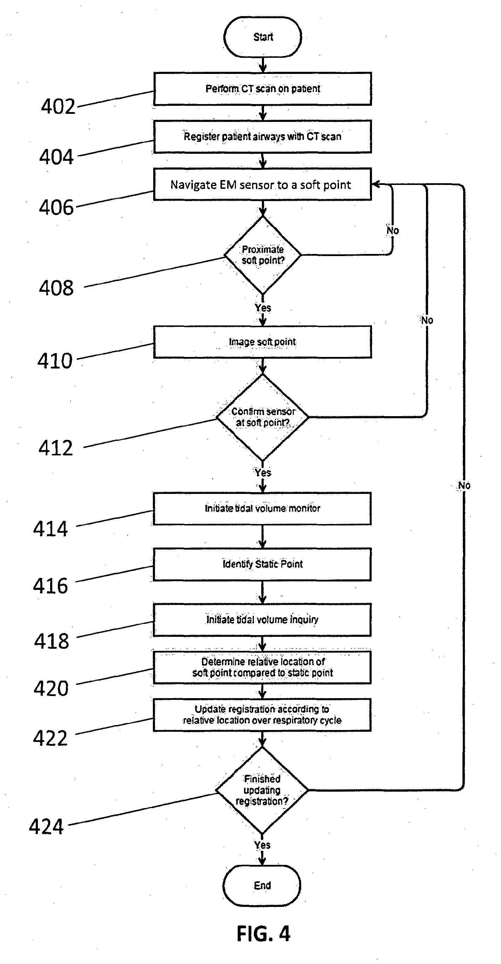

Referring now to FIG. 4, there is shown a flowchart of an example method for updating a registration of the 3D model with a patient's airways. As described above, at step 502, an area of interest, for instance the chest and lungs, of a patient is imaged using imaging methods such as, for example, a CT scan. At step 404, application 81 displays guidance for performing the registration of the CT scan with the patient's airways, as described above. During registration, the location of EM sensor 94 within the patient's airways is tracked, and a plurality of points denoting the location of EM sensor 94 within the EM field generated by EM generator 76 is stored.

At step 406, a physician or application 81 may identify one or more soft point targets, such as i.e. a nipple line, an esophagus, a rib outline, a secondary carina, etc. Application 81 begins the localized registration process by displaying guidance for navigating, through the a luminal network, through a wall of a luminal network, or percutaneously through a patient's skin, EM sensor 94 proximate to a soft point target, such as i.e. a nipple line, an esophagus, a rib outline, a secondary carina, etc., while tracking the location of EM sensor 94. In the alternative, by viewing a live video feed from a camera located proximate EM sensor 94 (e.g., in a bronchoscope) a soft point target may be detected visually by the clinician. Thereafter, at step 408, the clinician or application 81 may determine whether the sensor is located proximate to a determined soft point target. If the clinician or application 81 determines that EM sensor 94 is not proximate a soft point target, processing returns to step 406 where further guidance is displayed.

At step 410, the soft point target is imaged while EM sensor 94 is located proximate the soft point using, for example, CT imagining, cone beam CT imaging, or ultrasonic imaging. Using the image generated in step 410, at step 412, a clinician or application 81 may confirm EM sensor's 94 location at the soft point. If it is determined that the EM sensor 94 is not at the soft point target, processing returns to step 406 where further guidance is displayed. If EM sensor 94 is confirmed to be proximate the soft point, processing proceeds to step 414.

At step 414, the movement of the patient's chest caused by tidal volume breathing is sampled throughout one or more cycles of the patient's breathing cycle. Movement caused by tidal volume breathing may be sampled using one or more optical cameras positioned to view and record the movement of the patient's chest. The movement of the patient's chest may be used to estimate the movement caused by tidal breathing. In the alternative, sensors 74 may be sampled to determine the movement of the patient's chest during the patient's tidal breathing. The movement of the patient's chest sensed using sensors 74 similarly maybe be used to estimate the movement cause by tidal breathing.
At step 416, a clinician or application 81 may identify a static point, a point that moves minimally during a patient breathing cycle, such as, for example, a vertebral body, a main carina, thyroid cartilage, or an esophagus. Many of these static points will appear and will be cognizable and measureable on the initial CT scans and 3D generated model. Others may be monitored with sensors 74 placed on or near the identified static point. At step 418, the patient's tidal volume movement data is sampled throughout one or more cycles of the patient's breathing cycle.

At step 420, application 81 receives location data from EM sensor 94 throughout the patient's breathing cycle. The patient's breathing cycle is determined and monitored using the tidal volume monitor activated in step 414 and sampled in step 418. The location data from EM sensor 94 is converted into location data within the 3D model and compared to the location of the identified static point by application 81 to determine the location of the soft point relative to the static point throughout the breathing cycle. The relative location of the static point may be determined using, for example, triangulation. Potential methods of triangulation include, for example, direct linear transformation, mid-point determination of the Euclidean distance, essential matrix transformation, and optimal triangulation performed by determining the minimum-weight of various potential triangles from a set of points in a Euclidean plane. The relative soft point locations are stored as soft point data denoting the location of EM sensor 94.

At step 422, application 81 uses the soft point location data denoting the location of EM sensor 94 to perform localized registration to update the 3D model with the patient's airways proximate the soft point target. For example, localized registration may be performed based on a range of interpolation techniques, such as Thin Plates Splines (TPS) interpolation. In embodiments, TPS interpolation may be used for non-rigid registration of the points denoting the location of EM sensor 94 within the EM field generated by EM generator 76 stored during automatic registration with the 3D model, and may be augmented by additional points stored during localized registration.

At step 424, application 81 or a clinician determines if updating registration is complete if there are no remaining soft point targets for which localized registration has not been performed. If updating registration is not complete, processing returns to step 406, where application 81 displays guidance for navigating EM sensor 94 proximate the next soft point target. If updating registration is complete, the localized registration updating processing ends.

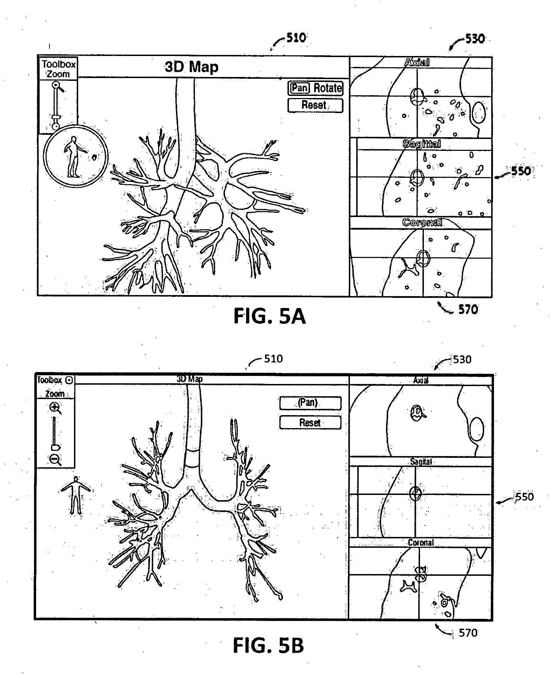

FIGS. 5A and 5B illustrate various windows that user interface 716 can present on the display 706 (FIG. 7) in accordance with embodiments of the present disclosure. Display 706 may present specific windows based on a mode of operation of the endoscopic navigation system 10, such as, for example, a target management mode, a pathway planning mode, and a navigation mode.

FIGS. 5A and 5B also illustrate the target management mode in accordance with embodiments of the present disclosure. After a target is identified, clinicians may review and manage to prioritize or confirm a location or size of each target. The target management mode may include a 3D map window 510 and three windows including the axial view window 530, the coronal view window 550, and the sagittal view window 570. The 3D map window 510 may be located on the left side and show a target 215. Three windows 530, 550, and 570 are selected based on the location of the target.

FIG. 5A shows a possible interface display after an initial registration. The initial registration allows for the physician to create a navigation plan to navigate to a soft spot near a treatment target. Upon reaching the soft spot and performing any of the localized registration methods described in FIGS 2-4 at the site, the 3D Map view 510, the axial view window 530, the coronal view window 550, and the sagittal view window 570 automatically update. FIG. 5B shows an updated display following a localized registration (FIG. 5A and 5B are shown in stark contrast merely for illustration purposes). As a physician navigates using any of the 2D or 3D displays 510, 530, 550, and 570, the displays further automatically update in order to present a stable image as the patient's chest moves during breathing cycles. The updating of the displays, as viewed from the perspective of the physician will remain unchanged, thus allowing the physician to navigate and apply treatment with a steady and accurate view.

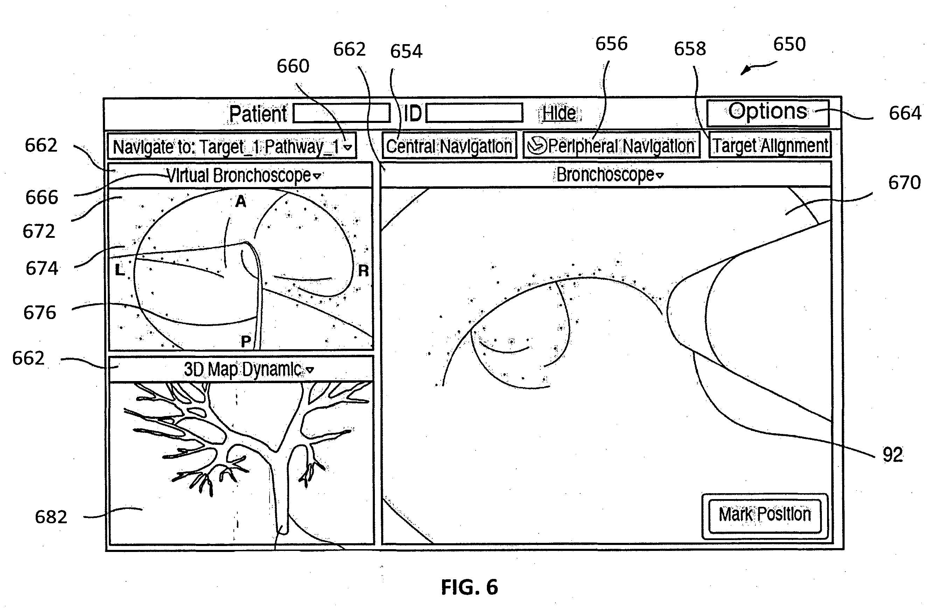

During navigation, user interface 716 (FIG. 7) may also present the physician with a view 650, as shown, for example, in FIG. 6. View 650 provides the clinician with a user interface for navigating to a target, such as a soft point target or a treatment target, including a central navigation tab 654, a peripheral navigation tab 656, and a target alignment tab 658. Central navigation tab 654 is primarily used to guide the bronchoscope 50 through the patient's bronchial tree. Peripheral navigation tab 456 is primarily used to guide the EWC 96, EM sensor 94, and LG 92 toward a target, including a soft point target and a treatment target. Target alignment tab 658 is primarily used to verify that LG 92 is aligned with a target after LG 92 has been navigated to the target using the peripheral navigation tab 656. View 650 also allows the clinician to select target 652 to navigate by activating a target selection button 660.

Each tab 654, 656, and 658 includes a number of windows 662 that assist the clinician in navigating to the soft point target. The number and configuration of windows 662 to be presented is configurable by the clinician prior to or during navigation through the activation of an "options" button 664. The view displayed in each window 662 is also configurable by the clinician by activating a display button 666 of each window 662. For example, activating the display button 666 presents the clinician with a list of views for selection by the clinician including a bronchoscope view 670, virtual bronchoscope view 672, 3D map dynamic view 682, MIP view (not shown), 3D map static view (not shown), sagittal CT view (not shown), axial CT view (not shown), coronal CT view (not shown), tip view (not shown), 3D CT view (not shown), and alignment view (not shown).

Bronchoscope view 670 presents the clinician with a real-time image received from the bronchoscope 50, as shown, for example, in FIG. 6. Bronchoscope view 670 allows the clinician to visually observe the patient's airways in real-time as bronchoscope 50 is navigated through the patient's airways toward a target.

Virtual bronchoscope view 672 presents the clinician with a 3D rendering 674 of the walls of the patient's airways generated from the 3D volume of the loaded navigation plan, as shown, for example, in FIG. 6. Virtual bronchoscope view 672 also presents the clinician with a navigation pathway 676 providing an indication of the direction along which the clinician will need to travel to reach a target. The navigation pathway 476 may be presented in a color or shape that contrasts with the 3D rendering 674 so that the clinician may easily determine the desired path to travel.

3D map dynamic view 682 presents a dynamic 3D model 684 of the patient's airways generated from the 3D volume of the loaded navigation plan. Dynamic 3D model 684 includes a highlighted portion 686 indicating the airways along which the clinician will need to travel to reach a target. The orientation of dynamic 3D model 684 automatically updates based on movement of the EM sensor 94 within the patient's airways to provide the clinician with a view of the dynamic 3D model 684 that is relatively unobstructed by airway branches that are not on the pathway to the target. 3D map dynamic view 682 also presents the virtual probe 679 to the clinician as described above where the virtual probe 679 rotates and moves through the airways presented in the dynamic 3D model 684 as the clinician advances the LG 92 through corresponding patient airways.

After performing any of the registration update methods shown in FIGS. 2-4, program 81 controls bronchoscope view 670, virtual bronchoscope view 672, 3D map dynamic view 682 according to the updated registration throughout the breathing cycle. As the patient's chest moves during breathing cycles, the updated registration accounts for the movement in order to show stable bronchoscope view 670, virtual bronchoscope view 672, and 3D map dynamic view 682. Stable views allow a clinician to navigate EWC 96, EM sensor 94, and LG 92 toward a treatment target or an additional registration target without continually disruptive chest movements causing as unstable view. Thus, the clinician is provided with more control and a simpler user experience navigating EWC 96, EM sensor 94, and LG 92 to the treatment target.

When a treatment target is reached, catheter biopsy tool 102 may be guided through EWC 96 and LG 92 so that treatment may be provided at the treatment target. While at the target, the improved localized registration allows for the target to be tracked more accurately in real time throughout the breathing cycle. As the procedure is carried out, the updated registration allows a physician to maintain treatment at the treatment target and avoid applying unwanted treatment on health tissue which may be adversely affected.

The improved localized registration additionally improves application of treatment to a treatment target outside of the airways. An access tool may be guided through EWC 96 or LG 92 to a location near a treatment or biopsy target. While near the treatment target, the improved localized registration allows for the target to be tracked more accurately through the walls of the tracheobronchial wall in real time throughout the breathing cycle. The improved tracking of the target reduces risk of increased damage to the bronchial tree when the access tool punctures the tracheobronchial wall to provide access to the treatment target.

The improved localized registration further aids percutaneous navigation and approach planning. The improved localized registration informs the location of the target as well as the location of other internal body features throughout the breathing cycle. A physician or application 81 may then determine a path for guiding a percutaneous needle to avoid puncturing internal body features while creating an accurate path to the treatment target to apply treatment throughout the breathing cycle.

Turning now to FIG. 7, there is shown a system diagram of workstation 80. Workstation 80 may include memory 702, processor 704, display 706, network interface 708, input device 710, and/or output module 712.

Memory 702 includes any non-transitory computer-readable storage media for storing data and/or software that is executable by processor 704 and which controls the operation of workstation 80. In an embodiment, memory 702 may include one or more solid-state storage devices such as flash memory chips. Alternatively or in addition to the one or more solid-state storage devices, memory 702 may include one or more mass storage devices connected to the processor 704 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 704. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by workstation 80.

Memory 702 may store application 81 and/or CT data 214. Application 81 may, when executed by processor 704, cause display 706 to present user interface 716. Network interface 708 may be configured to connect to a network such as a local area network (LAN) consisting of a wired network and/or a wireless network, a wide area network (WAN), a wireless mobile network, a Bluetooth network, and/or the internet. Input device 710 may be any device by means of which a clinician may interact with workstation 80, such as, for example, a mouse, keyboard, foot pedal, touch screen, and/or voice interface. Output module 712 may include any connectivity port or bus, such as, for example, parallel ports, serial ports, universal serial busses (USB), or any other similar connectivity port known to those skilled in the art.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

Detailed embodiments of such devices, systems incorporating such devices, and methods using the same are described above. However, these detailed embodiments are merely examples of the disclosure, which may be embodied in various forms. Therefore, specific structural and functional details disclosed herein are not to be interpreted as limiting but merely as a basis for the claims and as a representative basis for allowing one skilled in the art to variously employ the present disclosure in virtually any appropriately detailed structure. While the example embodiments described above are directed to the bronchoscopy of a patient's airways, those skilled in the art will realize that the same or similar devices, systems, and methods may also be used in other lumen networks, such as, for example, the vascular, lymphatic, and/or gastrointestinal networks.

## Claims

1. A navigation bronchoscopy system for registering an airway of a patient to a model of the airway, the system comprising:
a location sensor (94) capable of being navigated within the airway;
an electromagnetic field generator (76) configured to detect the location of the location sensor (94) as it is navigated within the airway;
an optical camera configured to view and record external movement of a patient's chest;
a display capable of displaying an image of the location sensor at a soft point that serves as a data point for a localized registration; and
a computing device (80) including a processor (704) and a memory (702) storing instructions which, when executed by the processor, cause the computing device to:
generate and display a model of the patient's airway based on images of the airway and determine a target within the model;
generate and display a pathway to the target and registration points;
track locations of the location sensor while the location sensor is navigated along the pathway to the registration points;
register the model airway to the airway based on the tracked locations of the location sensor arriving at the registration points;
generate and display a second pathway to a first soft point in the airway based on images of the airway, said soft point being selected from a nipple line, an esophagus, a rib outline and a secondary carina, said soft point serving as a data point for a localized registration;
track locations of the location sensor while the location sensor is navigated along the second pathway within the airway to the first soft point in the airway;
determine by imaging whether or not the location sensor is proximate the first soft point; wherein when it is determined that the location sensor is not proximate the first soft point, a representation of the location sensor, a representation of the airway in which the location sensor is placed and guidance for navigating the location sensor to the first soft point is displayed on the display; and
when it is determined that the location sensor is proximate the first soft point, the computing device generates patient tidal volume breathing movement data based on samples from the optical camera imaging movement of a patient's chest over a respiratory cycle to estimate movement caused by tidal breathing;
correlates the patient tidal volume breathing movement data with location sensor movement over a respiratory cycle;
updates the registration of the model of the airway to the airway based on the tracked location of the location sensor at the first soft point and based on the correlation of the patient tidal volume breathing movement data with location sensor movement over a respiratory cycle; and
determines whether or not updating the registration is complete; and
when it is determined that updating the registration is not complete, displays guidance for navigating the location sensor to a next soft point.

2. The system of claim 1, wherein the instructions, when executed by the processor, further cause the computing device to:
identify a known static point on the patient, the static point being a point that moves minimally during a patient breathing cycle; and
compare the location of the soft point to the static point.

3. The system of claim 2, wherein the instructions, when executed by the processor, further cause the computing device to:
update the registration of the airway model with the patient airway based on the comparison of the tracked location of the soft point to the static point.

4. The system of claim 2 or claim 3, wherein the static point is a vertebral body, a main carina, sternum, thyroid cartilage, rib or an esophagus.

5. The system of any preceding claim, wherein compared tracked location of the location sensor within the patient airway and the external patient motion are saved in a database to generate a predictive model according to patient characteristics.

## Patentansprüche

1. Navigationsbronchoskopiesystem zum Registrieren eines Luftwegs eines Patienten mit einem Modell des Luftwegs, wobei das System umfasst:
einen Positionssensor (94), der innerhalb des Luftwegs navigiert werden kann;
einen elektromagnetischen Feldgenerator (76), der ausgestaltet ist, um die Position des Positionssensors (94) zu detektieren, wenn er innerhalb des Luftwegs navigiert wird;
eine optische Kamera, die ausgestaltet ist, um externe Bewegung eines Brustkorbs eines Patienten zu betrachten und aufzuzeichnen;
eine Anzeige, die ein Bild des Positionssensors an einem Weichpunkt anzeigen kann, der als Datenpunkt für eine lokalisierte Registrierung dient; und
eine Rechenvorrichtung (80), die einen Prozessor (704) und einen Speicher (702) einschließt, der Anweisungen speichert, die bei Ausführung durch den Prozessor bewirken, dass die Rechenvorrichtung folgendes ausführt:
Generieren und Anzeigen eines Modells des Luftwegs des Patienten basierend auf Bildern des Luftwegs, und Bestimmen eines Zieles innerhalb des Modells;
Generieren und Anzeigen von einem Pfad zu dem Ziel und Registrierungspunkten;
Verfolgen von Positionen des Positionssensors, während der Positionssensor entlang des Pfads zu den Registrierungspunkten navigiert wird;
Registrieren des Modellluftwegs mit dem Luftweg basierend auf den verfolgten Positionen des Positionssensors, der an den Registrierungspunkten ankommt;
Generieren und Anzeigen eines zweiten Pfads zu einem ersten Weichpunkt in dem Luftweg basierend auf Bildern des Luftwegs, wobei der Weichpunkt ausgewählt ist aus einer Brustwarzenlinie, einem Ösophagus, einer Rippenkontur und einer Sekundärcarina, wobei der Weichpunkt als Datenpunkt für eine lokalisierte Registrierung dient;
Verfolgen von Positionen des Positionssensors, während der Positionssensor entlang des zweiten Pfads innerhalb des Luftwegs zu dem ersten Weichpunkt in dem Luftweg navigiert wird;
Bestimmen mittels Bildgebung, ob sich der Positionssensor in der Nähe des ersten Weichpunkts befindet oder nicht; wobei, wenn bestimmt wird, dass sich der Positionssensor nicht in der Nähe des ersten Weichpunkts befindet, eine Darstellung des Positionssensors, eine Darstellung des Luftwegs, in dem der Positionssensor platziert ist, und Anleitung zum Navigieren des Positionssensors zu dem ersten Weichpunkt auf der Anzeige angezeigt wird; und
wenn bestimmt wird, dass sich der Positionssensor in der Nähe des ersten Weichpunkts befindet, die Rechenvorrichtung Tidalvolumen-Atembewegungsdaten des Patienten basierend auf Abtastungen von der Bewegung eines Brustkorbs des Patienten durch Bildgebung mittels der optischen Kamera über einen Atemzyklus generiert, um die durch Tidalatmung verursachte Bewegung zu schätzen;
Korrelieren der Tidalvolumen-Atembewegungsdaten des Patienten mit der Positionssensorbewegung über einen Atemzyklus;
Aktualisieren der Registrierung des Modells des Luftwegs mit dem Luftweg basierend auf der verfolgten Position des Positionssensors an dem ersten Weichpunkt und basierend auf der Korrelation der Tidalvolumen-Atembewegungsdaten des Patienten mit der Positionssensorbewegung über einen Atemzyklus; und
Bestimmen, ob die Aktualisierung der Registrierung vollständig ist oder nicht; und
wenn bestimmt wird, dass die Aktualisierung der Registrierung nicht vollständig ist, Anzeigen von Anleitung zum Navigieren des Positionssensors zu einem nächsten Weichpunkt.

2. System nach Anspruch 1, wobei die Anweisungen bei Ausführung durch den Prozessor des Weiteren bewirken, dass die Rechenvorrichtung folgendes durchführt:
Identifizieren eines bekannten statischen Punkts auf dem Patienten, wobei der statische Punkt ein Punkt ist, der sich während eines Atemzyklus eines Patienten minimal bewegt; und
Vergleichen der Position des Weichpunkts mit dem statischen Punkt.

3. System nach Anspruch 2, wobei die Anweisungen bei Ausführung durch den Prozessor des Weiteren bewirken, dass die Rechenvorrichtung folgendes durchführt:
Aktualisieren der Registrierung des Luftwegmodells mit dem Patientenluftweg basierend auf dem Vergleich der verfolgten Position des Weichpunkts mit dem statischen Punkt.

4. System nach Anspruch 2 oder Anspruch 3, wobei der statische Punkt ein Wirbelkörper, eine Hauptcarina, das Sternum, Schilddrüsenknorpel, eine Rippe oder ein Ösophagus ist.

5. System nach einem der vorhergehenden Ansprüche, wobei die verglichene verfolgte Position des Positionssensors innerhalb des Patientenluftwegs und die externe Patientenbewegung in einer Datenbank gespeichert werden, um ein prädiktives Modell gemäß den Patientencharakteristika zu generieren.

## Revendications

1. Système de bronchoscopie de navigation pour repérer des voies respiratoires d'un patient sur un modèle des voies respiratoires, le système comprenant :
un capteur de localisation (94) capable de naviguer à l'intérieur des voies respiratoires ;
un générateur de champ électromagnétique (76) configuré pour détecter l'emplacement du capteur de localisation (94) lorsqu'il est déplacé à l'intérieur des voies respiratoires ;
une caméra optique configurée pour visualiser et enregistrer des mouvements externes du thorax du patient ;
un dispositif d'affichage capable d'afficher une image du capteur de localisation à un point mou qui sert de point de données pour un repérage localisé ; et
un dispositif informatique (80) comprenant un processeur (704) et une mémoire (702) stockant des instructions qui, lorsqu'elles sont exécutées par le processeur, amènent le dispositif informatique à :
générer et afficher un modèle des voies respiratoires du patient sur la base d'images des voies respiratoires et déterminer une cible à l'intérieur du modèle ;
générer et afficher une trajectoire vers la cible et les points de repérage ;
suivre des emplacements du capteur de localisation pendant que le capteur de localisation navigue le long de la trajectoire vers les points de repérage ;
repérer les voies respiratoires modèles pour les voies respiratoires sur la base des emplacements suivis par le capteur de localisation arrivant aux points de repérage ;
générer et afficher une deuxième trajectoire vers un premier point mou des voies respiratoires sur la base des images des voies respiratoires, ledit point mou étant choisi parmi une ligne de mamelon, un oesophage, un contour de côte et une carène secondaire, ledit point mou servant de point de données pour un repérage localisé ;
suivre des emplacements du capteur de localisation pendant que le capteur de localisation navigue le long de la deuxième trajectoire à l'intérieur des voies respiratoires jusqu'au premier point mou dans les voies respiratoires ;
déterminer par imagerie si le capteur de localisation est proche ou non du premier point mou ; lorsqu'il est déterminé que le capteur de localisation n'est pas proche du premier point mou, une représentation du capteur de localisation, une représentation des voies respiratoires dans lesquelles le capteur de localisation est placé et des conseils pour la navigation du capteur de localisation vers le premier point mou étant affichés sur le dispositif d'affichage ; et
lorsqu'il est déterminé que le capteur de localisation est proche du premier point mou, le dispositif informatique génère des données de mouvement de respiration de volume au repos du patient sur la base d'échantillons provenant de la caméra optique imageant le mouvement de la poitrine d'un patient au cours d'un cycle respiratoire afin d'estimer un mouvement causé par la respiration au repos ;
établit une corrélation entre les données de mouvement de respiration de volume au repos du patient et le mouvement du capteur de localisation au cours d'un cycle respiratoire ;
met à jour le repérage du modèle des voies respiratoires pour les voies respiratoires sur la base de l'emplacement suivi du capteur de localisation au premier point mou et sur la base de la corrélation des données de mouvement de respiration de volume au repos du patient avec le mouvement du capteur de localisation au cours d'un cycle respiratoire ; et
détermine si la mise à jour du repérage est terminée ou non ; et
lorsqu'il est déterminé que la mise à jour du repérage n'est pas terminée, affiche des conseils pour la navigation du capteur de localisation vers un prochain point mou.

2. Système selon la revendication 1, les instructions, lorsqu'elles sont exécutées par le processeur, amenant en outre le dispositif informatique à :
identifier un point statique connu sur le patient, le point statique étant un point qui bouge de manière minimale pendant le cycle respiratoire du patient ; et
comparer l'emplacement du point mou au point statique.

3. Système selon la revendication 2, les instructions, lorsqu'elles sont exécutées par le processeur, amenant en outre le dispositif informatique à :
mettre à jour le repérage du modèle des voies respiratoires avec les voies respiratoires du patient sur la base de la comparaison de l'emplacement suivi du point mou avec le point statique.

4. Système selon la revendication 2 ou selon la revendication 3, le point statique étant un corps vertébral, une carène principale, un sternum, un cartilage thyroïde, une côte ou un oesophage.

5. Système selon n'importe quelle revendication précédente, la localisation comparée du capteur de localisation à l'intérieur des voies respiratoires du patient et le mouvement externe du patient étant enregistrés dans une base de données afin de générer un modèle prédictif en fonction des caractéristiques du patient.
